# EUROPEAN PATENT SPECIFICATION

(11) **EP 4 114 261 B1**
(45) Date of publication and mention of the grant of the patent: **22.05.2024**
(21) Application number: 21714949.1
(22) Date of filing: 05.03.2021
(51) Int. Cl.: A61B 5/11, A61B 5/00, A61B 5/06

(54) **DETECTION DEVICE HOUSABLE OR INTEGRATED IN A FOOTWEAR**
IN EINEM SCHUHWERK UNTERBRINGBARE ODER INTEGRIERTE DETEKTIONSVORRICHTUNG
DISPOSITIF DE DÉTECTION POUVANT ÊTRE LOGÉ OU INTÉGRÉ DANS UNE CHAUSSURE

(30) Priority: 06.03.2020 IT 202000004810
(43) Date of publication of application: 11.01.2023
(73) Proprietor: ESTEPS S.R.L.S., 47923 Rimini (IT)
(72) Inventor: LOUHICHI, Nidhal, 47923 Rimini (IT)
(74) Representative: Gamba, Alessandro
(86) International application number: PCT/IB2021/051845
(87) International publication number: WO 2021/176408

(56) References cited:
- FR-A1- 3 072 271
- US-A1- 2015 340 928
- US-A1- 2017 135 444
- US-A1- 2018 122 211
- US-B2- 9 279 734

## Description

The present invention relates to a detection device suitable for detecting the posture and movement of a user. In particular, according to the present invention, such a detection device is housable in a footwear. Or in an embodiment variant, again according to the present invention, said detection device is integrated in a footwear.

The object of the present invention is also a system for analyzing the posture and movement, which comprises at least one detection device, preferably two detection devices, and at least one external electronic device, such as a PC, a smartphone or a tablet.

In further detail of the present invention, the detection device is specifically applied in the field of medicine (for example within the realm of medical rehabilitation) and/or sports.

Solutions of detection devices and footwear comprising specific detection devices, the purpose of which is to detect a series of information associated with the posture and/or movement of a user, are known from the prior art.

Such known solutions however have a plurality of problems, which have affected the diffusion and use thereof.

Firstly, the known solutions are not able to constantly detect the desired information. Conventionally, the known products have a series of problems associated with the supply of electricity thereof, and therefore with the duration of the detections.

Secondly, the known solutions furthermore are particularly cumbersome, particularly with reference to the "electrical component" thereof. The greater the dimensions, the more the usability and aesthetics of the footwear for which they are provided, are affected. Document FR 3 072 271 A1 relates to a system for analyzing and quantifying e.g. posture of a user wearing a shoe, which has communication units configured such that electronic box of the sole transmits user information to an external terminal and to an electronic box of another sole.

It is therefore the object of the present invention to have a detection device which is housable or integrable in a footwear which meets the aforesaid needs of the field by providing an alternative to the known solutions, thus resolving the specific problems thereof.

In other words, the object of the present invention is to provide a detection device which ensures a constant and long-lasting detection of the posture and/or movement of a user so as to provide a complete picture, i.e. having an increased amount of information. In this way, the increased quantity of information collected may be taken advantage of by a treating physician or a trainer, but also simultaneously by the user him/herself according to needs.

According to the present invention, such an object is achieved by a detection device according to claim 1. Simultaneously, such an object is achieved by a system for analyzing posture and movement according to claim 6. Preferred embodiments of the invention are defined in the dependent claims.

The features and advantages of the above invention will become apparent from the following description, given by way of non-limiting example, according to the accompanying figures, in which:

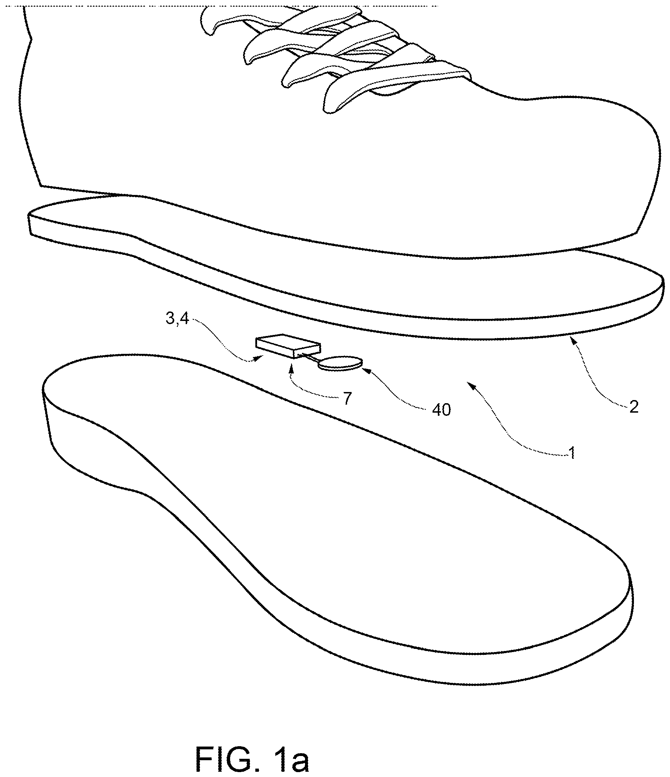

- Figure 1a shows a view with separate parts, of a footwear comprising a sole in which the detection device according to a preferred embodiment of the present invention is housed;

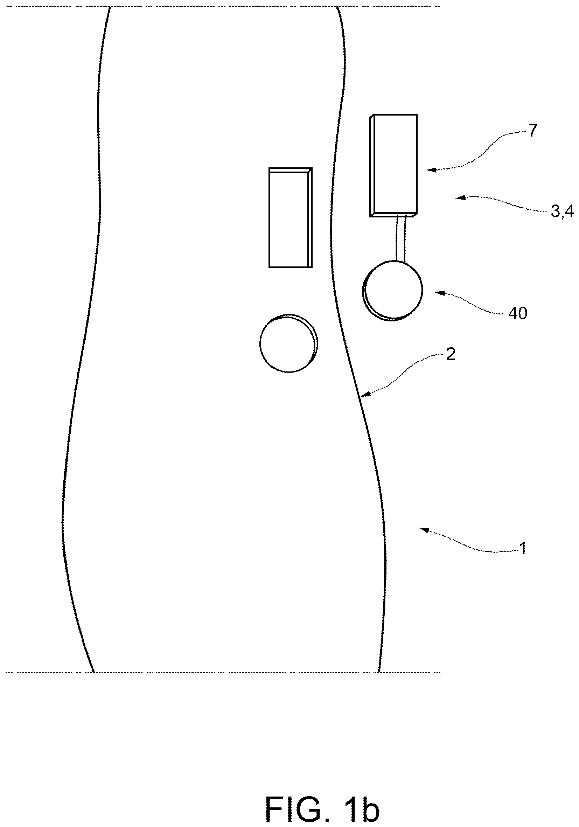

- Figure 1b shows a sectional view of the footwear in Figure 1a;

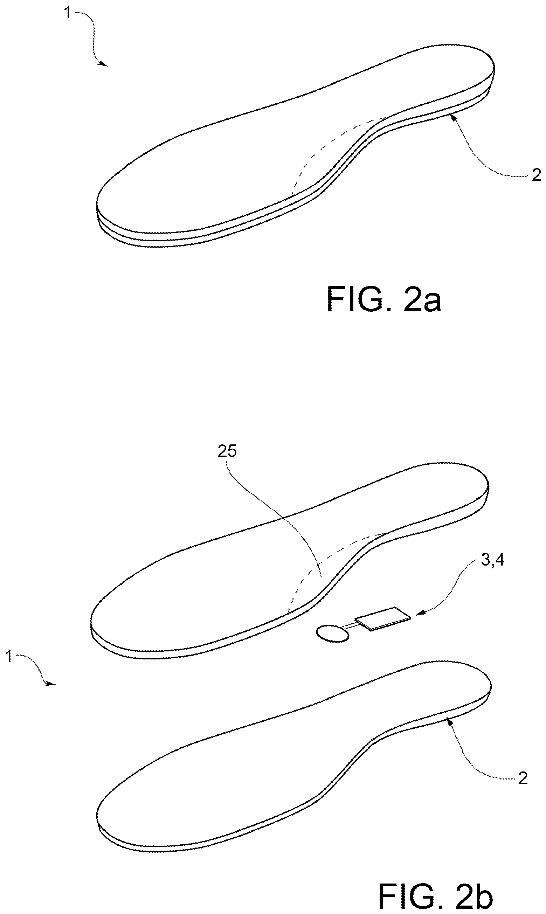

- Figure 2a shows a perspective view of the detection device in a preferred embodiment of the present invention, i.e. in the shape of an insole;
- Figure 2b is a perspective view, with separate parts, of the detection device in Figure 2a;

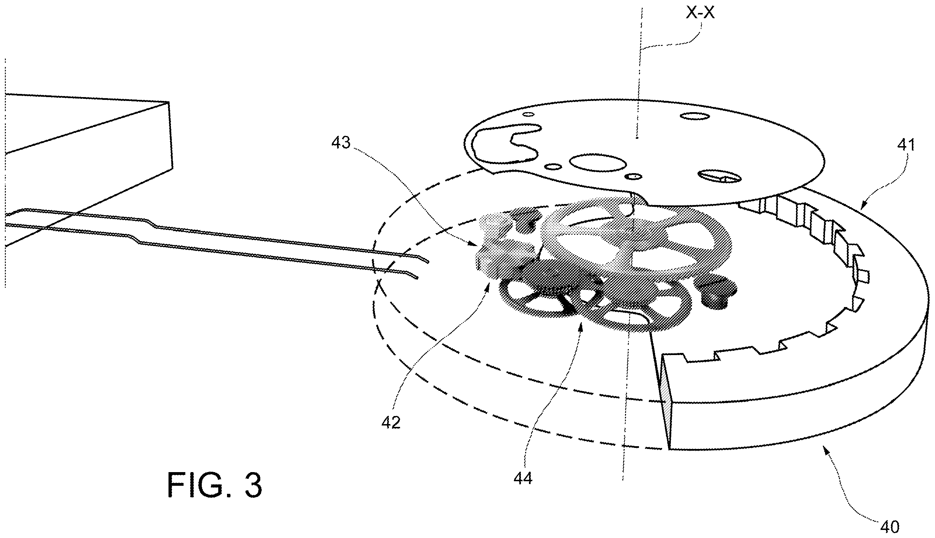

- Figure 3 is a perspective view, with separate parts, of some components comprised in the detection device according to the present invention;

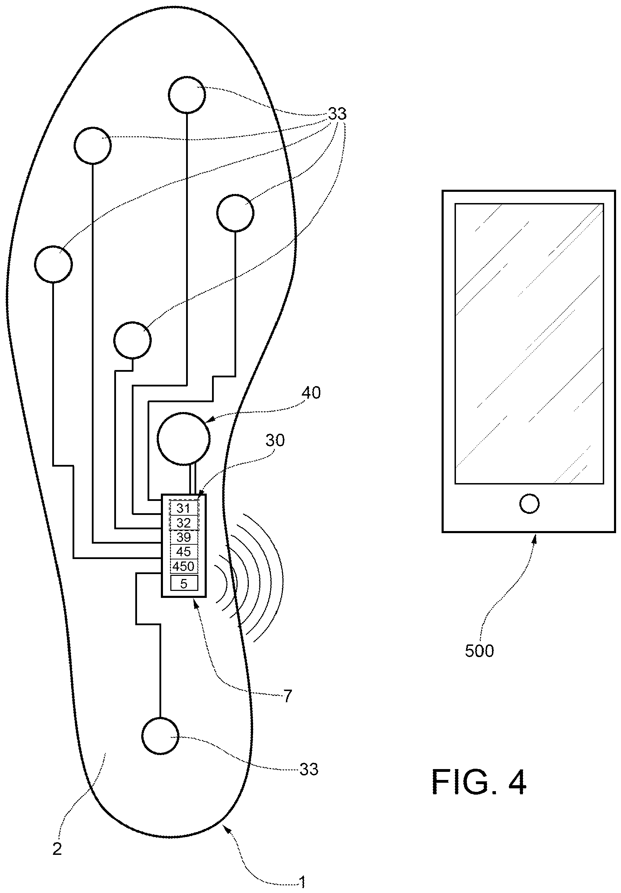

- Figure 4 shows a diagrammatical view of a system for analyzing the posture and movement of a user according to a preferred embodiment;

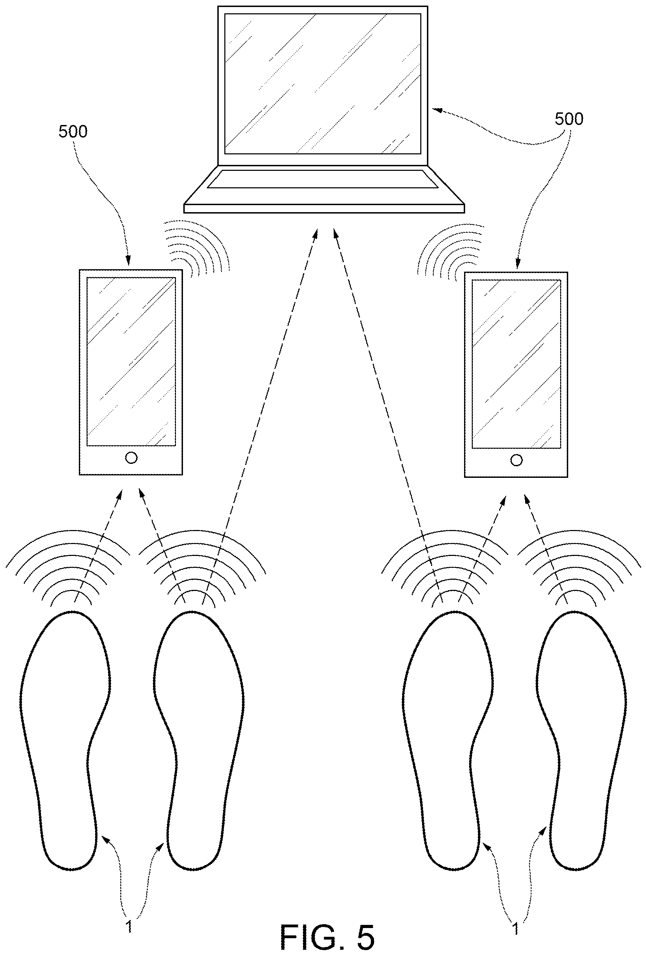

- Figure 5 shows a diagrammatical view of a system for analyzing the posture and movement of a user according to a further preferred embodiment.

According to the accompanying drawings, a detection device as a whole according to the present invention is indicated by reference numeral 1.

According to the present invention, the detection device 1 is suitable for being housed in a footwear.

Or, according to an embodiment variant of the present invention, the detection device 1 is integrated in a footwear.

It should be noted that the term footwear in the present invention is not intended as limited to any embodiment. Indeed, footwear means any type of garment suitable for protecting the foot of the user who is to perform a given activity.

Preferably, the footwear may be a shoe or a boot. According to the type of footwear, the detection device is suitable for detecting both the static and dynamic methods of use of the user.

In the present disclosure, detecting "posture" identifies static-type detections, while detecting "movement" identifies dynamic-type detections.

According to the present invention, the detection device 1 comprises a main body 2 which is insertable in a footwear.

According to a preferred embodiment, the main body 2 is a sole or insole which is insertable in the footwear.

According to the present invention, the detection device 1 comprises a main body 2 which is integrated in a footwear.

According to a preferred embodiment, the main body 2 is a sole or insole which is integrated in the footwear.

Preferably, with the exception of the features described below, the sole or insole does not have limiting features for the invention.

According to further embodiments, the main body 2 is integrated in other portions of the footwear, for example in the upper.

According to the present invention, the detection device 1 comprises a detection group 3 embedded in the main body 2.

Preferably, the specific object of the detection group 3 is to detect static and dynamic features of the user.

According to the present invention, the detection group comprises at least one detection member 30 comprising at least one accelerometer element 31 and at least one gyroscope element 32.

Preferably, the accelerometer element 31 is suitable for detecting certain dynamic features of motion.

Preferably, the accelerometer element 31 is of the type having three axes.

Preferably, the accelerometer element 31 is of the low-power type.

Preferably, the gyroscope element 32 is suitable for detecting both static features and dynamic features. In particular, the gyroscope element 32 is suitable for detecting the position, in particular a given angle of inclination, of the foot of the user. In particular, the gyroscope element 32 is suitable for detecting the direction of a movement of the foot of the user.

Preferably, the gyroscope element 32 is of the type having three axes.

Preferably, the gyroscope element 32 is of the low-power type.

According to the present invention, the accelerometer element 31 and the gyroscope element 32 are suitable for simultaneously constantly detecting, in such a way as to have the data of both detections for each moment in time.

According to a preferred embodiment, the detection member 30 further comprises a pressure measuring element 33, preferably a plurality of pressure measuring elements 33, which are suitable for detecting the presence of the user and/or the pressure the user puts on the detection device 1.

According to a preferred embodiment, each pressure measuring element 33 is of the type comprising resistive pressure sensors.

Preferably, each pressure measuring element 33 is thin, substantially extending planarly without thickness.

According to a preferred embodiment, the pressure measuring element 33 extends in such a way as to be positionable at all the homogeneous parts of the sole of the foot.

According to a preferred embodiment, the main body 2 (preferably in the sole or insole embodiment thereof) comprises pressure measuring elements 33 which are strategically positioned to have a sensor for each preferred area of the foot, in particular one in the heel, one in the outer plantar arch and four in the front area of the sole of the foot proximal to the toes.

According to a preferred embodiment, the accelerometer element 31, the gyroscope element 32 and the pressure measuring element 33 are suitable for simultaneously constantly detecting over time so as to have the data relating to each detection for each moment in time.

According to the present invention, the detection device 1 further comprises a power supply group 4 embedded in the main body 2.

The object of the power supply group 4 is to supply electricity to the detection group 3. Indeed, according to the present invention, the power supply group 4 is operatively connected to the detection group 3.

Preferably, the power supply group 4 comprises:
- a generator organ of alternating current 40, comprising a rotary element 41 suitable for rotating freely when subjected to movement;
- a generator organ of direct current 45, which is electrically connected to the generator organ of alternating current 40 that receives the alternating current and transforms it into direct current in order to supply electricity to the detection group 3.

According to the present invention, the power supply group 4 is as close as possible to the detection group 3.

Preferably, the generator organ of alternating current 40 is in a position which is proximal to the detection group 3.

According to a preferred embodiment, the generator organ of direct current 45 also comprises a battery element 450 suitable for storing the direct electrical current in order to pass it to the detection group 3.

Preferably, the battery element 450 is suitable for supplying electricity to the detection group 3 according to needs, regardless of the movement of the generator organ of alternating current 40.

According to a preferred embodiment, the detection group 3, the generator organ of direct current 45 are in a single box-like element 7 embedded in the main body 2.

According to a preferred embodiment, the generator organ of alternating current 40 is substantially disc-shaped, comprising a main axis X-X and an imaginary plane on which it extends substantially orthogonally to the main axis X-X.

Preferably, the generator organ of alternating current 40 has the substantial dimensions of a coin.

According to a preferred embodiment, the rotary element 41 has an eccentric shape with respect to said main axis X-X about which it rotates, in which said main axis X-X extends substantially vertically and/or substantially horizontally. Preferably, the positioning of the main axis X-X is relative to the ground surface on which the user moves. According to a preferred embodiment, said main axis X-X is inclined by a few degrees with respect to the vertical.

According to a preferred embodiment, the generator organ of alternating current 40 comprises a rotor 42 and a stator 43.

Preferably, rotor 42 is operatively connected to said rotary element 41.

According to a preferred embodiment, the generator organ of alternating current 40 further comprises transmission means 44 connecting the rotary element 41 with rotor 42.

According to a preferred embodiment, the detection device 1 further comprises a receiving/transmitting data group 5, which is operatively connectable to an external electronic device 500, such as a smartphone or a tablet.

Preferably, also the receiving/transmitting data group 5 is embedded in the main body 2.

Preferably, said operatively connected receiving/transmitting data group 5 is in turn supplied with electricity by the power supply group 4.

According to a preferred embodiment, the detection group 3 also comprises a storage unit 39 suitable for storing the data detected by the detection member 30.

According to a preferred embodiment, the detection group 3 and the power supply group 4, and preferably also the receiving/transmitting data group 5, are embedded in the main body 2 in a central/side position.

Preferably, the detection group 3 and the power supply group 4, and preferably also the receiving/transmitting data group 5, are positioned in an area which is substantially proximal to the plantar arch of the user of the sole or insole.

Indeed, the main body 2 in the shape of sole or insole preferably comprises a plantar arch reinforcement body 25, in which the detection group 3 and the power supply group 4 and the receiving/transmitting data group 5 are housed.

According to a preferred embodiment, said plantar arch reinforcement body 25 faces the inside of the foot of the user.

As mentioned, the object of the present invention is also a system for analyzing the posture and movement of a user, comprising at least one detection device 1, preferably two detection devices 1, and an external electronic device 500 that is operatively connectable to said detection device 1.

According to a preferred embodiment, the system for analyzing posture and movement comprises a pair of footwear, in which each footwear is inserted or integrated in a detection device 1.

Preferably, the external electronic device 500 is configured to receive data from each detection device 1 and is configured to correlate such information.

According to a preferred embodiment, the external electronic device 500 is a smartphone and/or a tablet and/or a workstation.

Preferably, the detection device 1 is suitable for transmitting information simultaneously to several external electronic devices 500.

According to a preferred embodiment, each external electronic device 500 is configured with specific programs (or apps) for collecting and managing the information detected by each detection device 1.

According to a preferred embodiment, the main body 2 in the form of insole comprises a preferably removable coating film suitable for protecting the insole and in certain cases the footwear itself, against the action of sweat, liquids and bacterial attacks.

Innovatively, the detection device according to the present invention achieves the preset object.

Advantageously, the detection device is suitable for constantly detecting data in such a way as to provide them to the user and/or a physician.

Advantageously, the supply of electricity is ensured by virtue of the self-powering of the detection device itself.

Advantageously, the battery serves to store a quantity of power such as to also ensure the supply of electricity to the detection device also in the absence of movements. Preferably, the detection device is suitable for sending the data collected also in the absence of movements, and therefore of self-powering.

Advantageously, the detection device is not very cumbersome and is versatile.

Advantageously, the detection device is housable or easily integrable in a footwear.

Advantageously, the detection device in the "insole" version is removable from a footwear to be insertable in another footwear.

Advantageously, the physician/trainer takes advantage of a constant flow of information from one or more users/patients, by means of the detection device.

Advantageously, the physician/trainer is free to do his/her work while operating remotely, without the need to be in the presence of the user.

Advantageously, the physician/trainer receives a very broad statistical sample of information.

Advantageously, the physician/trainer is able to guide the rehabilitation or physical training in light of any specific detections.

Advantageously, possible injuries to the lower limbs are easy to anticipate.

Advantageously, the user him/herself is able to monitor his/her daily routine and/or sports activities.

Advantageously, the status of the footwear is also monitorable, and for example the end of the life cycle thereof.

Advantageously, the status of the insole is also monitorable. Advantageously, the "electronic component" of the device is removable from the insole to be mountable in a new, specially-shaped insole.

Advantageously, the "mechanical component" of the detection device, i.e. the member that generates the alternating current, is housed in a clear housing which allows visually checking for possible malfunctions thereof.

It is apparent that those skilled in the art may make changes to the above-described invention in order to meet contingent and specific needs, all contained within the scope of protection defined by the following claims.

## Claims

1. A detection device (1) for detecting the posture and movement of a user, comprising:
i) a main body (2), wherein the main body (2) is an insole insertable into a footwear;
ii) a detection group (3) that is embedded in the main body (2) and comprises at least one detection member (30) comprising at least one accelerometer element (31) and at least one gyroscope element (32);
iii) a power supply group (4) that is embedded in the main body (2) and is operatively connected to the detection group (3) in order to supply electricity thereto, wherein the power supply group (4) comprises:
- a generator organ of alternating current (40) substantially disc-shaped, comprising a main axis (X-X), an imaginary plane on which it extends substantially orthogonally to the main axis (X-X), and comprising a rotary element (41) having an eccentric shape with respect to said main axis (X-X), suitable for rotating freely when subjected to movement, wherein said main axis X-X is inclined by a few degrees with respect to the vertical, wherein the generator organ of alternating current (40) comprises a rotor (42) and a stator (43), wherein said rotor is operatively connected to said rotary element (41);
- a generator organ of direct current (45), which is electrically connected to the generator organ of alternating current (40) that receives the alternating current and transforms it into direct current in order to supply electricity to the detection group (3).

2. Detection device (1) according to claim 1, wherein the generator organ of direct current (45) also comprises a battery element (450) suitable for storing the direct electrical current in order to pass it to the detection group (3).

3. Detection device (1) according to any one of the preceding claims, further comprising a group for receiving/transmitting data (5), which is embedded in the main body (2) and is operatively connectable to an external electronic device (500), such as a smartphone or a tablet, wherein said group for receiving/transmitting data (5) that is operatively connected to an electronic device is, in turn, supplied with electricity by the power supply group (4).

4. Detection device (1) according to any one of the preceding claims, wherein the detection group (3) and the power supply group (4) are embedded in the main body (2) in a central/lateral position so as to be aligned with the plantar arch of the user of the insole.

5. Detection device (1) according to claim 4, wherein the insole comprises a plantar arch reinforcement body (25), in which the detection group (3) and the power supply group (4) are housed.

6. A system for analyzing the posture and movement of a user, comprising at least one detection device (1) according to any one of the preceding claims, and an external electronic device (500) that is operatively connectable to said detection device (1).

7. System for analyzing posture and movement according to claim 6, comprising a pair of footwear, wherein a detection device (1) is inserted in each footwear.

8. System for analyzing posture and movement according to claims 6 and 7, wherein the external electronic device (500) is configured to receive data from each detection device (1) and is configured to correlate this information.

## Patentansprüche

1. Erfassungsvorrichtung (1) zur Erfassung der Haltung und Bewegung eines Benutzers, aufweisend:
i) einen Hauptkörper (2), wobei der Hauptkörper (2) eine Einlegesohle ist, die in einen Schuhartikel einlegbar ist;
ii) eine Erfassungsgruppe (3), die in den Hauptkörper (2) eingebettet ist und zumindest ein Erfassungselement (30) aufweist, das zumindest ein Beschleunigungsmesser-Element (31) und zumindest ein Gyroskop-Element (32) aufweist;
iii) eine Stromversorgungsgruppe (4), die in den Hauptkörper (2) eingebettet ist und mit der Erfassungsgruppe (3) operativ verbunden ist, um Strom an diese zuzuführen, wobei die Stromversorgungsgruppe (4) aufweist:
- eine Einrichtung zur Erzeugung von Wechselstrom (40), die im Wesentlichen scheibenförmig ist und eine Hauptachse (X-X), eine gedachte Ebene, auf der sie sich im Wesentlichen orthogonal zu der Hauptachse (X-X) erstreckt, und ein Drehelement (14) mit einer außermittigen Form in Bezug auf die Hauptachse (X-X) aufweist, welches, wenn es Bewegung ausgesetzt wird, frei drehen kann, wobei die Hauptachse (X-X) um ein paar Grad in Bezug auf die Vertikale geneigt ist, wobei die Einrichtung zur Erzeugung von Wechselstrom (40) einen Rotor (42) und einen Stator (43) aufweist, wobei der Rotor mit dem Drehelement (41) operativ verbunden ist;
- eine Einrichtung zur Erzeugung von Gleichstrom (45), die mit der Einrichtung zur Erzeugung von Wechselstrom (40) elektrisch verbunden ist und die den Wechselstrom aufnimmt und diesen in Gleichstrom umwandelt, um der Erfassungsgruppe (3) Strom zuzuführen.

2. Erfassungsvorrichtung (1) nach Anspruch 1, wobei die Einrichtung zur Erzeugung von Gleichstrom (45) ferner ein Batterieelement (450) aufweist, das zur Speicherung des Gleichstroms geeignet ist, um diesen an die Erfassungsgruppe (3) weiterzuleiten.

3. Erfassungsvorrichtung (1) nach einem der vorstehenden Ansprüche, ferner aufweisend eine Gruppe zum Empfangen/Senden von Daten (5), die in den Hauptkörper (2) eingebettet ist und mit einer externen elektronischen Vorrichtung (500), zum Beispiel einem Smartphone oder einem Tablet, operativ verbindbar ist, wobei die mit einer elektronischen Vorrichtung operativ verbundene Gruppe zum Empfangen/Senden von Daten (5) wiederum durch die Stromversorgungsgruppe (4) mit Strom versorgt wird.

4. Erfassungsvorrichtung (1) nach einem der vorstehenden Ansprüche, wobei die Erfassungsgruppe (3) und die Stromversorgungsgruppe (4) derart in einer zentralen/lateralen Position in den Hauptkörper (2) eingebettet sind, dass sie mit dem Plantarbogen des Nutzers der Einlegesohle ausgerichtet sind.

5. Erfassungsvorrichtung (1) nach Anspruch 4, wobei die Einlegesohle einen Plantarbogen-Verstärkungskörper (25) aufweist, in dem die Erfassungsgruppe (3) und die Stromversorgungsgruppe (4) aufgenommen sind.

6. System zur Analyse der Haltung und Bewegung eines Benutzers, aufweisend zumindest eine Erfassungsvorrichtung (1) nach einem der vorstehenden Ansprüche sowie eine externe elektronische Vorrichtung (500), die mit der Erfassungsvorrichtung (1) operativ verbindbar ist.

7. System zur Analyse der Haltung und Bewegung nach Anspruch 6, aufweisend ein Paar Schuhartikel, wobei eine Erfassungsvorrichtung (1) in jedem Schuhartikel eingesetzt ist.

8. System zur Analyse der Haltung und Bewegung nach den Ansprüchen 6 und 7, wobei die externe elektronische Vorrichtung (500) eingerichtet ist, Daten von jeder Erfassungsvorrichtung (1) zu empfangen, und eingerichtet ist, diese Informationen zu korrelieren.

## Revendications

1. Dispositif de détection (1) permettant de détecter la posture et le mouvement d'un utilisateur, comprenant :
i) un corps principal (2), où le corps principal (2) est une semelle intérieure pouvant être insérée dans une chaussure ;
ii) un groupe de détection (3) qui est intégré dans le corps principal (2) et comprend au moins un membre de détection (30) comprenant au moins un élément accéléromètre (31) et au moins un élément gyroscope (32) ;
iii) un groupe d'alimentation électrique (4) qui est intégré dans le corps principal (2) et est fonctionnellement connecté au groupe de détection (3) afin de lui fournir de l'électricité, le groupe d'alimentation électrique (4) comprenant :
- un organe générateur de courant alternatif (40) sensiblement en forme de disque, comprenant un axe principal (X-X), un plan imaginaire sur lequel il s'étend sensiblement orthogonalement à l'axe principal (X-X), et comprenant un élément rotatif (41) ayant une forme excentrique par rapport audit axe principal (X-X), adapté pour tourner librement lorsqu'il est soumis à un mouvement, où ledit axe principal (X-X) est incliné de quelques degrés par rapport à la verticale, où l'organe générateur de courant alternatif (40) comprend un rotor (42) et un stator (43), où ledit rotor est fonctionnellement connecté audit élément rotatif (41) ;
- un organe générateur de courant continu (45), qui est connecté électriquement à l'organe générateur de courant alternatif (40) qui reçoit le courant alternatif et le transforme en courant continu pour fournir de l'électricité au groupe de détection (3).

2. Dispositif de détection (1) selon la revendication 1, dans lequel l'organe générateur de courant continu (45) comprend également un élément de batterie (450) adapté pour stocker le courant électrique continu afin de le passer dans le groupe de détection (3).

3. Dispositif de détection (1) selon l'une quelconque des revendications précédentes, comprenant en outre un groupe de réception/transmission de données (5), qui est intégré dans le corps principal (2) et peut être fonctionnellement connecté à un dispositif électronique externe (500), tel qu'un smartphone ou une tablette, ledit groupe de réception/transmission de données (5) qui est fonctionnellement connecté à un dispositif électronique étant, à son tour, alimenté en électricité par le groupe d'alimentation électrique (4).

4. Dispositif de détection (1) selon l'une quelconque des revendications précédentes, dans lequel le groupe de détection (3) et le groupe d'alimentation électrique (4) sont intégrés dans le corps principal (2) dans une position centrale/latérale de manière à être alignés avec la voûte plantaire de l'utilisateur de la semelle intérieure.

5. Dispositif de détection (1) selon la revendication 4, dans lequel la semelle intérieure comprend un corps de renfort de voûte plantaire (25), dans lequel sont logés le groupe de détection (3) et le groupe d'alimentation électrique (4).

6. Système permettant d'analyser la posture et le mouvement d'un utilisateur, comprenant au moins un dispositif de détection (1) selon l'une quelconque des revendications précédentes, et un dispositif électronique externe (500) qui peut être fonctionnellement connecté audit dispositif de détection (1).

7. Système permettant d'analyser la posture et le mouvement selon la revendication 6, comprenant une paire de chaussures, dans lequel un dispositif de détection (1) est inséré dans chaque chaussure.

8. Système permettant d'analyser la posture et le mouvement selon les revendications 6 et 7, dans lequel le dispositif électronique externe (500) est configuré pour recevoir des données de chaque dispositif de détection (1) et est configuré pour corréler ces informations.
